# EUROPEAN PATENT APPLICATION

(11) **EP 2 858 017 A1**
(43) Date of publication of application: **08.04.2015**
(21) Application number: 13187028.9
(22) Date of filing: 02.10.2013
(51) Int. Cl.: G06Q 10/08

(54) **Method and system for monitoring a perishable product**

(71) Applicant: Bio Trace Trading Ltd., D14 Dublin (IE)
(72) Inventor: The designation of the inventor has not yet been filed
(74) Representative: Lucey, Michael

(57) **Abstract**

A method and a system for monitoring a perishable product in a container are disclosed. A plurality of contactless data transmitters are provided, each encoded with a respective unique identifier. A corresponding plurality of containers are provided, each adapted to be filled with and to contain a perishable product, and to each of which a respective encoded contactless data transmitter is secured. Each filled container is stored, in at least one facility. Data defining at least one characteristic of the perishable product filling a container and at least one temporal reference for the filling operation is associated with the unique identifier. Movement and/or use of the container is monitored over a respective predetermined time interval elapsing from the at least one temporal reference associated with the unique identifier, by reading the unique identifier from the contactless data transmitter.

## Description

### Field of the Invention

The invention relates to a method for monitoring a perishable product in a container, and to a system embodying the method. More particularly, the invention relates to a method and system for monitoring the movement and causing the timely use of blood packs across a network of storage and/or medical facilities.

### Background of the Invention

Collection, storage and logistical methods have long been devised to ensure that a perishable product can be brought to a point of use as shortly after its collection or manufacture as possible, then kept thereat under optimal storage conditions adapted to maximise its useable or 'shelf' life, until it is either used or discarded after expiry of a predetermined period of time. Many different industries, including for instance the fresh produce retail industry and the pharmaceutical and healthcare industry, have long used a wide variety of corresponding systems, of which the expressions 'use-by-date' or 'sell-by-date', that are stamped to innumerable products, have become synonymous.

In the specific context of healthcare, typically blood is collected from donors through blood donations, stored in resilient containers commonly referred to as packs, and eventually used in blood transfusions for relevant patients. Collected blood constitutes a perishable product which has a limited useable life and requires refrigerated storage. Typically, venous blood is collected, then stored either as whole blood or separated by centrifugation into red blood cells in solution, the latter being the most commonly used blood product. Units of whole blood and units of red blood cells are both kept refrigerated in substantially the same temperature range of 1 to 6 °C, however whole blood units have a maximum permitted storage period of 35 days, whereas red blood cells units have a longer corresponding period of 42 days. It is also known to store blood platelets, however these have a very much shorter shelf life of 7 days maximum, as no longer-term storage solutions have yet been devised. Platelets are typically stored at room temperature at approximately 22 °C and subjected to frequent or continuous agitation.

The supply of blood packs from collection points to storage facilities, typically blood banks at or in close proximity to healthcare dispensaries, such as hospitals, is habitually performed according to storage capacity and historical use, on the premise that e.g. a large regional hospital uses significantly more blood packs per unit of time than a local clinic. Besides conventional inventory management systems based on manual stock-taking and data inputting, no systems are known for coordinating and optimising the transfer of blood packs from one facility to another, for instance when a large-scale emergency may require additional blood supply from remote blood banks. In such instances, ad hoc requests for blood packs are made, typically by phone and with a significant administrative burden.

Conventional inventory management systems in use for blood supply management are known to be based upon manual data input, fraught with format or outright technical incompatibilities, costly to install and operate, and thus difficult to scale. Such systems generally require a high level of manual intervention to monitor stocks and manage ad hoc transfers, and are entirely passive where the perishable character of each blood pack is concerned: namely, such systems carry out simple inventory functions based on a 'First-In-First-Out' (FIFO) inventory management approach, and do not actively attempt to maximise use of the blood stock registered therein or therewith during its useful life. The large variability which exists in storage results for different donors, combined with limited quality testing procedures in still many facilities, impedes efforts to determine reliable and consistent indicators of quality for blood products.

In the above context, recent medical research has begun to investigate whether a blood pack's age, relative to its time of collection, is a factor in transfusion efficacy, i.e. whether older blood nearing its 35 or 42 days end-of-life increases medical risks for the recipient. Results have been conflicting and inconsistent to date, yet length of storage is the main variable on the basis of which to estimate blood quality loss. Accordingly, some recent approaches to blood storage and management have attempted to balance a reduction of the average blood product age at the time of its transfusion with maintaining sufficient availability of in-date products, effectively running the above-described FIFO approach in parallel with a smaller-scale 'Last-In-First-Out'(LIFO) approach.

Nevertheless, all such newer approaches still exhibit the same shortcomings as the conventional inventory management systems, in that they still require a high level of manual intervention to monitor stocks and manage transfers remain, and lack a real time indicator apt to prioritise use or transfer of blood products according to their age. In the case where minimum stocks are exceeded an automated redistribution may be required. A new and improved method and system are therefore required, which mitigate at least some of the above shortcomings of the prior art.

### Summary of the Invention

The present invention provides a network-distributed solution for storing and monitoring a perishable product in a container, to which an identifier transmitter or electronically read identifier such as a barcode is secured for enabling the capture, tracking and updating in real time of data representative of the perishable product characteristics and scheduled expiry. Advantageously, this solution maximises the efficient use of stored perishable product by mitigating wastage that habitually results from non-use by expiry. For example, the solution may automatically generate and broadcast alert messages about the impending expiry of a perishable product stored at a location and, in the absence of any timely use at that location subsequent to the alert, the solution may automatically transfer (prompt or organise) the perishable product to another location at which it is more likely to be used before its expiry.

Accordingly, in a first aspect of the present invention, a method for storing a perishable product is provided, which comprises the steps of encoding a respective unique identifier in one ore more contactless data transmitters ; securing each encoded contactless data transmitter to a respective container adapted to contain the perishable product ; filling one or more containers with the perishable product ; storing the one or more filled container in at least one facility ; for each filled container, associating, with at least one data processing terminal, data defining at least one characteristic of the perishable product filling the container and at least one temporal reference for the container-filling step with the unique identifier; and monitoring, at the at least one terminal, movement and/or use of each filled container over a respective predetermined time interval elapsing from the at least one temporal reference by reading the unique identifier from the contactless data transmitter.

In a further advantage the invention provides and manages product transfer and redistribution to reduce waste and improve utilisation/usage. It will be appreciated that reasons why product may be transferred/redistributed include Shelf life; Demand; Over supply (minimum stocks exceeded); or an emergency situation. Product maybe shipped regardless of shelf life, for example for demand or over supply in a particular location the invention transfers/redistributes the product to maximise product utilisation.

In an embodiment of the method according to the invention, the at least one terminal may be a first network-connected terminal, wherein the step of associating comprises the further steps of inputting the at least one characteristic of the perishable product and the at least one temporal reference at a second network-connected data processing terminal remote from the first terminal, and communicating the input data with the unique identifier from the second terminal to the first terminal over the network.

In an embodiment of the method according to the invention, the step of monitoring may comprise the further step of periodically querying the respective contactless data transmitter of one or more containers stored in the at least one facility and updating a database at the at least one terminal.

An embodiment of the method according to the invention may comprise the further step of causing the at least one terminal to automatically output a message about a container stored in the at least one facility, when at least a first portion of the predetermined time interval associated with that container is reached.

A variant of this embodiment of the method may comprise the further step of causing the at least one terminal to automatically output a container transfer message for transferring the container to an alternative facility for use, when a second portion of the predetermined time interval associated with that container is reached.

In a variant of this further embodiment of the method, the predetermined time interval may be bounded by the temporal reference and a use-by-date, the second portion of the predetermined time interval may be reached before the use-by-date, and the first portion of the predetermined time interval may be reached before the second portion.

For any of the above embodiments of the method according to the invention, and still others, the perishable product may be blood, whereby the at least one characteristic may be a blood type and the at least one temporal reference may be a blood collection date. The primary blood products which will be managed are Whole Blood, RBCs and Platelets.

The method effectively enables each perishable product unit in a supply chain to actively monitor itself, via the interaction of the contactless data transmitter and the managing software affixed thereto with the data processing terminal, which validates and updates data specific to the perishable product through the unique identifier carried by its respective container. Users are alerted to any issues relevant to the perishable product, for instance variations in the storage temperature, unauthorised movement from the storage facility, (breach of minimum stock levels) and still more events deducible from the data capture of the unique identifier and optionally one or more relevant sensors of the storage facility.

Users are also alerted, at least once or sequentially, to the impending expiry of the perishable product, as the temporal reference is monitored against the predetermined time interval and periods thereof. In the absence of any use of the perishable product after one or more such alerts, the method also enables a perishable product unit to effectively transfer itself to another facility, for instance one graded higher than the current facility in terms of characteristics-matching units used per unit of time, without further input or validation from an operator, under a relevant failsafe rule triggered by proximity with the end of life of the product. Regardless of the extent and complexity of the possible permutations of all of these implementation variants, the contactless data transmitter associated with each perishable product unit effectively enables full traceability of the unit over time, from collection to use or disposal.

According to another aspect of the present invention, there is also provided a system for storing a perishable product, comprising one or more contactless data transmitters, each encoded with a respective unique identifier ; one or more containers, each adapted to be filled with and to contain a perishable product, and to the or each of which an encoded contactless data transmitter is respectively secured ; storage means of at least one facility for storing the or each filled container ; means for associating data defining at least one characteristic of the perishable product contained in a container and at least one temporal reference for the container-filling operation with the unique identifier; monitoring means for monitoring movement and/or use of each filled container over a respective predetermined time interval elapsing from the at least one temporal reference ; and means for reading the unique identifier from the contactless data transmitter.

In an embodiment of the system according to the invention, the means for associating may comprise a first networked-connected data processing terminal, the monitoring means may comprise a second network-connected data processing terminal remote from the first terminal, wherein the first terminal is configured to input the at least one characteristic of the perishable product and the at least one temporal reference, and to communicate the input data with the unique identifier to the second terminal over the network.

In an embodiment of the system according to the invention, the reading means may be further adapted to periodically query the respective contactless data transmitter of one or more containers stored in the at least one facility and updating a database at the at least one terminal.

In an embodiment of the system according to the invention, the monitoring means may be further configured to automatically output a respective message about a container stored in the at least one facility, when at least a first portion of the predetermined time interval associated with that container is reached.

In a variant of this embodiment of the system, the monitoring means may be further configured to automatically output a container transfer message for transferring the container to an alternative facility for use, when a second portion of the predetermined time interval associated with that container is reached.

In a variant of this further embodiment of the system, the predetermined time interval may be bounded by the temporal reference and a use-by-date, the second portion of the predetermined time interval may be reached before the use-by-date, and the first portion of the predetermined time interval may be reached before the second portion.

For any of the above embodiments of the method according to the invention, and still others, the contactless data transmitter may be selected from the group comprising radio-frequency identification (RFID) passive tags and labels, battery-assisted passive RFID tags and labels, RFID active tags and labels, RuBee tags and Memory Spot tags. Barcode

According to a further aspect of the present invention, there is also provided a set of instructions recorded on a data carrying medium which, when read from the medium and processed by a data processing terminal operably connected with a network, configures the terminal to associate data defining at least one characteristic of a perishable product filling a container stored in at least one facility and at least one temporal reference for the container-filling operation with a unique identifier encoded in a contactless data transmitter secured to the container , monitor movement and/or use of the container over a respective predetermined time interval elapsing from the at least one temporal reference, and read or receive the unique identifier from each contactless data transmitter.

Other aspects of the invention are as described herein.

### Brief Description of the Drawings

For a better understanding of the invention and to show how the same may be carried into effect, there will now be described by way of example only, specific embodiments, methods and processes according to the present invention with reference to the accompanying drawings in which:
Figure 1 illustrates a typical environment in which the method and system of the invention may be embodied, including a plurality of facilities, a plurality of contactless data transmitters secured to respective blood packs at one or more of the facilities, and a plurality of networked terminals interfaced with contactless data transmitter readers at one or more of the facilities ;
Figure 2 is a logical diagram of a typical hardware architecture of the terminals shown in Figure 1 ;
Figure 3 provides a functional overview of a system according to the invention in the environment of Figure 1, embodied as a database and a plurality of data processing modules distributed across the terminals of Figures 1 and 2 ;
Figure 4 is a flowchart representation of a first embodiment of the data processing logic of the system of Figure 3, including steps of registering and monitoring blood packs over time ;
Figure 5 is a flowchart representation of the data processing logic constituting the monitoring step of Figure 4 ;
Figure 6 is a flowchart representation of a second embodiment of the data processing logic of the system of Figure 3, further comprising networked communications between remote terminals ; and
Figure 7 is a flowchart representation of a third embodiment of the data processing logic of the system of Figure 3, wherein the step of monitoring further comprises monitoring storage conditions of the blood packs over time.

### Detailed Description of the Drawings

There will now be described by way of example a specific mode contemplated by the inventors. In the following description numerous specific details are set forth in order to provide a thorough understanding. It will be apparent however, to one skilled in the art, that the present invention may be practiced without limitation to these specific details. In other instances, well known methods and structures have not been described in detail so as not to unnecessarily obscure the description.

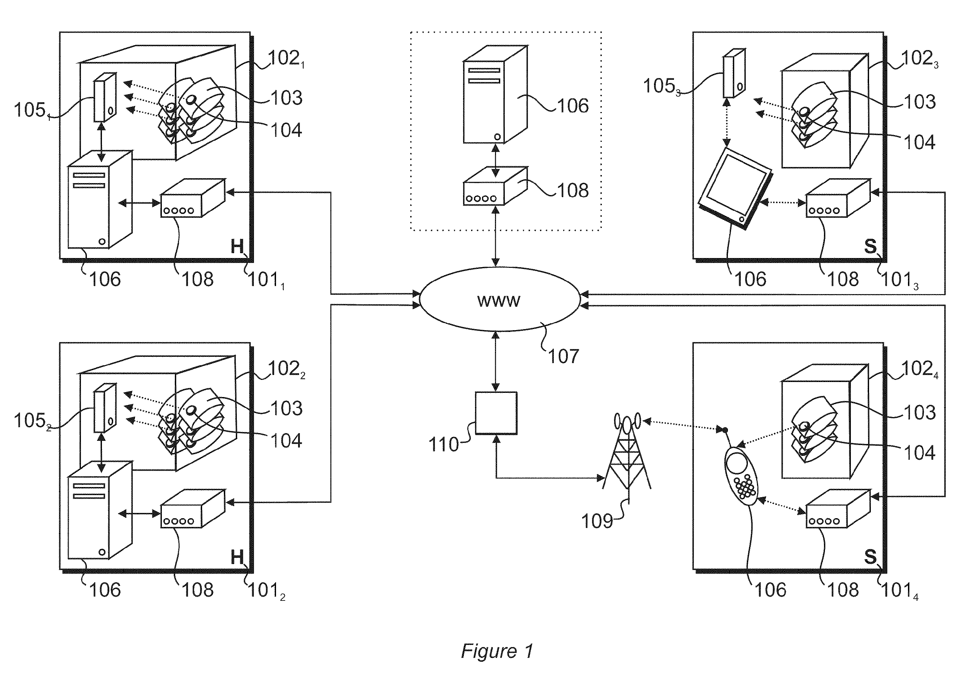

With reference to Figures 1 to 3 herein, an example embodiment of a system according to the invention is shown within an environment comprising a plurality of healthcare facilities 101_{N}. A first facility 101₁ is a regional hospital, a second facility 101₂ is a local hospital, a third facility 101₃ is a small doctor surgery and a fourth facility 101₄ is an ad hoc blood collection venue. Each facility 101_{N} is configured with a temperature - controlled blood storage location 102_{N,} the scale of which corresponds to the blood volume handling requirements of the facility over time, thus wherein the blood storage location 102₁ of the regional hospital 101₁ habitually stores more blood packs 103 than the blood storage location 102₂ of the local hospital 101₂ and substantially more than the blood storage location 102₃ of the small doctor surgery 101₃.

Each blood pack 103 is a resilient pouch made of a clear inert elastomer, sealable after filling with blood according to known techniques. According to the invention, each blood pack 103 further comprises a contactless data transmitter 104 secured thereto, which may be any of a radiofrequency identification (RFID) passive tag, a battery-assisted passive RFID tag, an RFID active tag, a RuBee tag or a Memory Spot tag, (Barcode) wherein each contactless data transmitter 104 has a unique identifier encoded therein, for instance an alphanumerical sequence constituting a unique serial number.

Correspondingly, the blood storage location 102_{N} of each healthcare facility 101_{N} is equipped with means 105 for receiving and decoding the unique identifier from each contactless data transmitters 104 stored therein, and communicating the decoded unique identifier to a respective data processing terminal 106. In the case of the hospital facilities 101₁, 101₂ at which the blood storage location 102_{N} is relatively large, for instance a blood bank in a dedicated room, such means is one or more wall-mounted RFID readers 105_{N} located adjacent the blood packs 103 and/or any access to the storage room. In the case of the much smaller doctor surgery 101₃, at which the blood storage location 102₃ is for instance a consumer fridge device 102₃, such means is a wirelessly-networkable near field communication (NFC)-RFID reader 105 located adjacent, or simply resting on top of, the fridge device. In the case of the ad hoc blood collection venue 101₄, wherein the equipment for setting it up must be compact, easily transportable and likewise easily deployable, such means is a personal communication device 106, typically a smartphone, the conventional components of which are configurable ad hoc as a NFC-RFID reader 105 with a relevant set of instructions. Thus, each of the RFID readers 105 is either operably interfaced with a data processing terminal 106, whether by wire as in the case of the two hospitals 101₁, 101₂ or wirelessly as in the case of the doctor surgery 101₃, or integral therewith as in the case of the RFID-enabled smartphone 106.

Each data processing terminal 106 is also connected to at least one communication network 107, in the example a Wide Area Network permitting data communication between all such data processing terminals 106, of which the Internet 107 is an example.

The smartphone 106 has wireless telecommunication emitting and receiving functionality over a cellular telephone network configured according to the International Mobile Telecommunications-4000 ('IMT - 4000') network industry standards, thus including UMTS/CDMA-4000/EDGE or '3G', IEEE802.16e/LTE Advanced or '4G', and wherein telecommunication is performed as voice, alphanumeric or audio-video data using the Short Message Service ('SMS') protocol, the Wireless Application protocol ('WAP') the Hypertext Transfer Protocol ('HTTP') or the Secure Hypertext Transfer Protocol ('HTTPS').

In the cellular telephone network, the signal is relayed respectively to or from the smartphone 106 by the geographically-closest communication link relay 109 of a plurality thereof, which routes digital signals to communication target(s) by means of a remote gateway via a MSC or base station 110. Alternatively, or additionally, the smartphone 106 may have wireless telecommunication emitting and receiving functionality over a wireless local area network ('WLAN') conforming to the 802.11 standard (Wi-Fi™). In the WLAN, telecommunication is likewise performed as voice, alphanumeric and/or audio-video data using the Internet Protocol (IP), Voice data over IP ('VoIP') protocol, Hypertext Transfer Protocol ('HTTP') or Secure Hypertext Transfer Protocol ('HTTPS'), the signal being relayed respectively to or from the smartphone 106 by a wireless (WLAN) router 108 interfacing it with the WAN communication network 107.

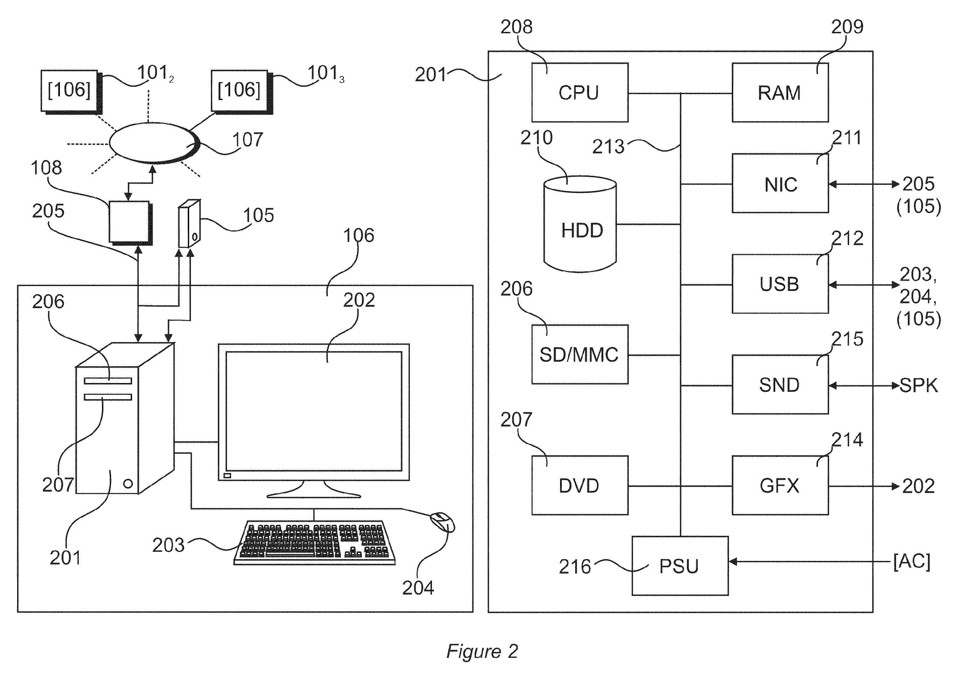

Other data processing terminals 106 of the environment emit and receive data encoded as a digital signal over a wired data transmission conforming to the IEEE 802.3 ('Gigabit Ethernet') standard and/or a wireless local area network ('WLAN') conforming to the 802.11 standard (Wi-Fi™), wherein the signal may be relayed respectively to or from the data processing terminals 106 via, respectively, a wired and/or wireless router 108 again interfacing them with the WAN communication network 107. Generally, the data processing terminals 106 may be any portable or desktop data processing device having data processing means and storage means, apt to be interfaced or configured with a RFID reader 105 as previously described. Accordingly, a typical hardware architecture of a data processing terminal 106 is now shown in Figure 2 in further detail, by way of non-limitative example.

The data processing terminal 106 is typically a computer configured with a data processing unit 201, data outputting means such as video display unit (VDU) 202, and data inputting means such as HiD devices, commonly a keyboard 203 and a pointing device (mouse) 204, as well as the VDU 202 itself if it is a touch screen display, in addition to the RFID reader 105. The computer is also configured with data inputting/outputting means such as a wired or wireless network connection 205 to a data communication network 108, 107 and to the RFID reader 105 if it is a network-enabled device, a magnetic data-carrying medium reader/writer 206 and an optical data-carrying medium reader/writer 207.

Within the data processing unit 201, a central processing unit (CPU) 208 provides task co-ordination and data processing functionality. Sets of instructions and data for the CPU 208 are stored in memory means 209 and a hard disk storage unit 210 facilitates non-volatile storage of the instructions and the data. A network interface card (NIC) 211 provides the interface to the network connection 205. A universal serial bus (USB) input/output interface 212 facilitates connection to the keyboard and pointing devices 203, 204 and to the RFID reader 105 if it is a USB-compliant device.

All of the above components are connected to a data input/output bus 213, to which the magnetic data-carrying medium reader/writer 206 and optical data-carrying medium reader/writer 207 are also connected, in addition to a video adapter 214, which receives CPU instructions over the bus 213 for further processing and outputting analogue or digital image data to VDU 202, and an audio adapter or soundcard 215, which receives CPU instructions over the bus 213 for further processing and outputting analogue sound data to speakers or the like (not shown). All the components of data processing unit 201 are powered by a power supply unit 216, which receives electrical power from a local mains power source and transforms same according to component ratings and requirements.

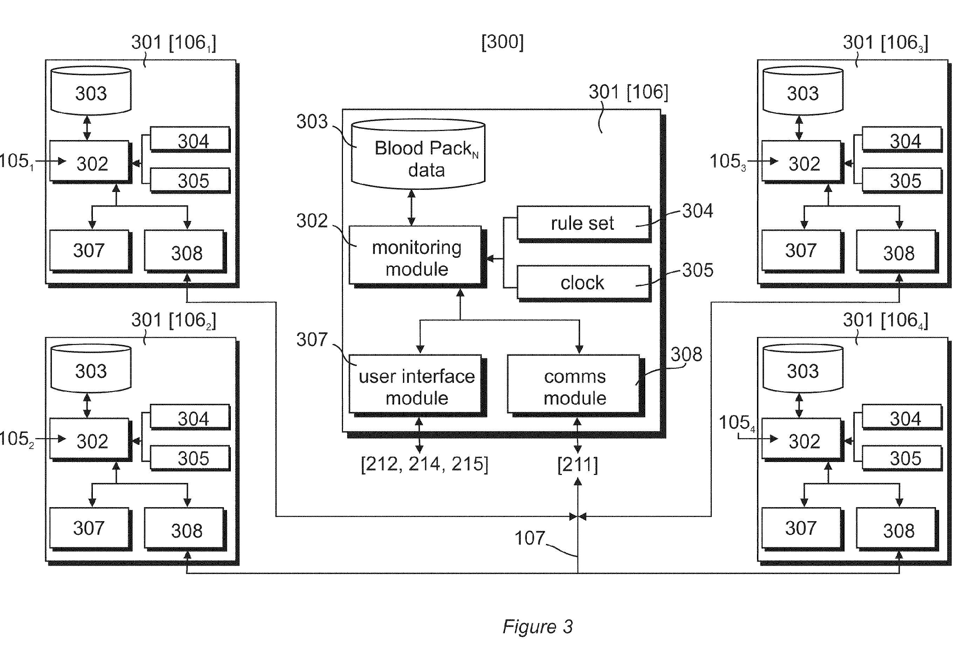

An embodiment of a system 300 according to the invention in the environment of Figure 1 is now shown in Figure 3, embodied as networked sub-systems 301 processed and distributed across the terminals 106, each sub-system 301 including a monitoring data processing module 302, a database 303, a rule set module 304, a timing module 305, a user interface module 307 and a communication module 308, all operably configured for registering and monitoring stored blood packs 103 as described hereafter. The system 300 described hereafter is adapted to register and monitor stored blood packs 103 both locally at each terminal 106 _{N} of each facility 101_{N} and remotely at a terminal 106 configured as a coordinating server.

When a blood pack 103 is first stored at a facility subsequently to a blood collection, the unique identifier encoded in the contactless data transmitter 104 secured thereto is read and decoded by an RFID reader 105 and communicated to the monitoring data processing module 302. The monitoring module 302 stored the unique identifier in the database 303, along with at least one further characteristic for the blood pack contents, and at least one temporal reference. The characteristic is preferably at least the type (A, B, O) and rhesus (positive, negative) of the blood contained therein, input through the user interface module 307 with the keyboard 203 via the USB interface 212. The temporal reference is for instance a timestamp representative of the time at which the container was filled with blood, again input through the user interface module 307, or representative of the time at which the blood pack unique identifier was received from the RFID reader 105, and input to the monitoring module 302 by a clock module 305. The clock module 305 is synchronised with remote clock modules 305 of remote sub-systems 301 via the communications module 308, in order to avoid time discrepancies across the system 300.

For any blood pack 103 stored at the facility, the monitoring module 302 continually monitors its availability for local use in a medical procedure against its predetermined expiry, by reference to one or more predetermined time intervals embedded in a rule set module 304, to the database-stored temporal reference which defines the start of the one or more predetermined intervals applicable to that blood pack 103, and to the current time provided by the clock module 305.

A core predetermined time interval is bounded by the temporal reference and an end date representative of the expiry of the blood contained in a blood pack 103, i.e. the date by which this blood may not be used in any medical procedure any more, as biologically unfit for purpose by that time. The time interval may therefore be 35 or 42 days calculated from the temporal reference, depending upon the blood product type in the container 103, i.e. whole blood or red blood cells respectively.

The rule set module 304 defines several portions, or sub-intervals, within the above core time interval. A first portion, constituted for instance by half of the core time interval, causes the monitoring module 302 to output a message about the stored blood pack 103 which has reached half of its useful life, either to the user interface module 307 or to the communications module 308 or both, for prompting a terminal user at the facility 101 at which this blood pack 103 is stored, to prioritize its use relative to other, newer blood packs 103. The user interface module 307 outputs an audible warning signal via the sound card 215 and/or a display warning message via the graphics card 214. Alternatively, or additionally, the communications module 308 of the central server 106 sends a network warning message to the sub-system 301 of the facility 101_{N} at which the half-expired blood pack 103 is stored, whereat the message again causes the local user interface module 307 to output an audible warning signal via the sound card 215 and/or a display warning message via the graphics card 214.

A second portion, constituted for instance by two thirds of the core time interval, causes the monitoring module 302 to again output a message about the stored blood pack 103 which has reached two thirds of its useful life, either to the user interface module 307 or to the communications module 308 or both, this time for instructing a terminal user at the facility 101 at which this blood pack 103 is stored, to transfer it to an alternative facility 101_{N} at which it is more likely to be used before its expiry date is reached. This rule is expected to be triggered most frequently in relation to blood packs 103 stored at facilities at which their use is relatively infrequent, typically doctor surgeries 101₃, the transfer of which would be for the benefit of relatively intense blood pack users, typically a hospital 101₁, 101₂ with an accident & emergency department. The user interface module 307 outputs a display transfer message via the graphics card 214 and, for a terminal 106 suitable equipped and configured, also causes a relevant container and/or routing order to be printed out, complete with the unique identifier of the relevant blood pack 103 to ensure the correct container is shipped.

The communications module 308 of the central server 106 sends a network transfer message to the sub-system 301 of the facility 101_{N} at which the two thirds-expired blood pack 103 is stored, whereat the message again causes the local user interface module 307 to output a display transfer message via the graphics card 214 and, for a terminal 106 suitable equipped and configured, also causes a relevant container and/or routing order to be printed out, complete with the unique identifier of the relevant blood pack 103 to ensure the correct container is shipped.

The rule set module 304 may also provide for the dynamic adjustment of the time interval and sub-intervals thereof according to a variety of environmental variables affecting stored blood packs 103, which may be monitored by the monitoring module 302 suitably interfaced with one or more relevant sensors. A highly-relevant environmental variable is constituted by variations in the temperature of the storage, e.g. a raised temperature relative to the accepted optimal range of 1 to 6 °C over a sufficient period accelerates the biological degradation of the stored blood. In this case, the respective core time interval for each stored blood pack 103 affected by the variation, regardless of its respective temporal reference, may be decremented proportionally to the temperature increase.

The database 303 may store further characteristics for the blood pack contents, for instance input at the sub-system 301 processed at the terminal 106₄ of the ad hoc collection facility 101₄ and representative of any one or more of the collecting location and conditions, donor characteristics, collected volume and the like. Such further characteristics are uploaded to at least the database 303 of the sub-system 301 processed by the central server 106, and may be distributed to the respective databases 303 of each sub-system 301 across the network for improving data redundancy across the entire system 300.

The embodiment described hereinbefore is considered to provide the best level of operational security and redundancy to the system 300, as it ensures that registration and monitoring of stored blood packs 103 continues at each facility 101_{N} irrespective of any interruption in network communications, unexpected unavailability of a network 107, 109, 110, scheduled or emergency maintenance of the coordinating server 106 and similar events. Accordingly, it will be readily understood by the skilled reader that many alternative configurations of the system 300 may be devised and embodied without difficulty, and without departing from the inventive principles described herein.

For instance, overall data storing and processing resources required to implement and sustain the functionality of the system 300 may be reduced by configuring the central server with a sub-system 301 as previously described, thus inclusive of the database 303, but limiting the configuration of the distributed sub-system 301 at each terminal 106 of a healthcare facility 101 to at least the user interface module 307, the communication module 308 and an RFID reader Application Programmer Interface (API) interfacing the terminal 106_{N} with a local RFID reader 105, sufficient in functional terms to input a blood pack unique identifier at the time of collection and/or storage and communicate same to the central server 106, and to output timely alarm or blood pack transfer messages received from the central server 106.

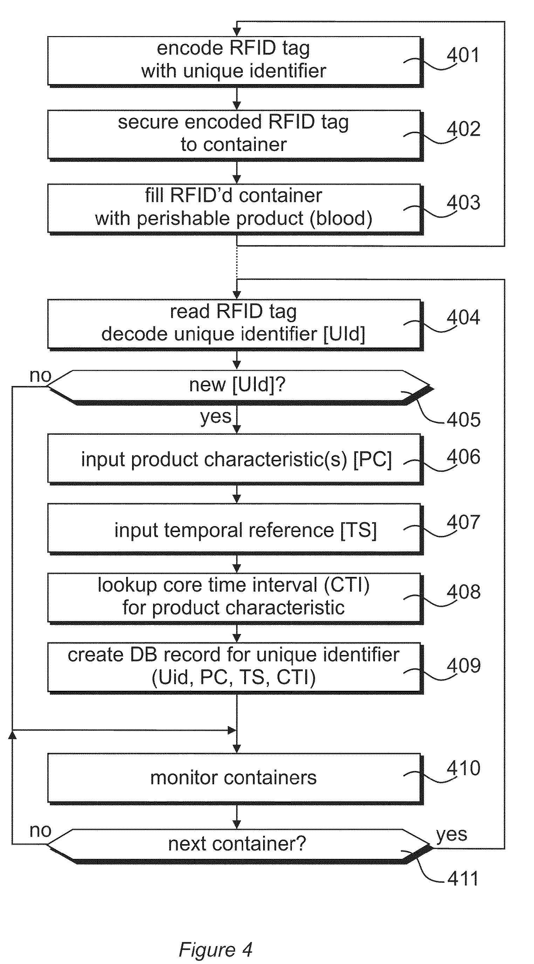

With reference now to Figure 4, a simplified representation of the data processing steps performed by the system 300 for storing, registering and monitoring blood packs 103 is provided.

At a first step 401, the RFID tag 104 is encoded with a unique identifier, for instance an alphanumerical sequence. The sequence may be devised so that a first portion thereof, for instance the first digit, commonly identifies the product as either whole blood or red blood cells; so that a second portion thereof, for instance the second digit, commonly identifies a core characteristic of the blood product such as it group; so that a third portion thereof, for instance the third digit, commonly identifies a another characteristic of the blood product such as it rhesus, and so on and so forth, provided always that a sufficient number of digits serves to uniquely distinguish each blood pack 103 from all others stored at all facilities 101_{N} of the system.

At step 402, the encoded RFID tag is secured to the container 103 and, at step 403, the RFI'd container is eventually filled with blood. It will be readily understood by the skilled reader that the sequential order of steps 401 to 403 is essentially immaterial, and may be dictated by such variables as the blood collecting parameters and procedure, the core principle being that each filled blood pack 103 carries an RFID tag encoded with a unique identifier apt to uniquely identify the blood pack within the entire system 300.

The RFID filled blood pack 103 is presented for registration into the system 300 as soon as practicable, for instance at the terminal 106₄ of the ad hoc blood collecting venue 101₄. At step 404, the RFID tag 104 of the presented blood pack 103 is read by the RFID reader 105 interfaced with the terminal 106 and the unique identifier is decoded and input to the monitoring module 302. A first question is asked at step 405, as to whether the decoded unique identifier is a new unique identifier not yet stored in the database 303. The question is answered in the affirmative for the newly-presented blood pack 103 and, at step 406, the at least one product characteristic is input to the monitoring module 302 and the temporal reference is next input to the monitoring module 302 at step 407. It will be readily understood that steps 406 and 407 may be easily automated, when one or more portions of the unique identifier are representative of the product characteristics as previously described and by querying the clock module 305 for a timestamp.

In this embodiment, the sub-system 301 stores and processes the database 303 and performs the monitoring locally whereby, at step 408, the monitoring module 302 invokes the rule set module 304 to look up and calculate the core time interval for the input product characteristic, for instance 35 days if the product characteristic is whole blood. At step 409, the monitoring module 302 creates a new database record in the database 303 for the new unique identifier, which includes the unique identifier, the product characteristic, the temporal reference and the determined core time interval. Control proceeds to a monitoring step 410, as is the case when the question of step 405 is answered negatively.

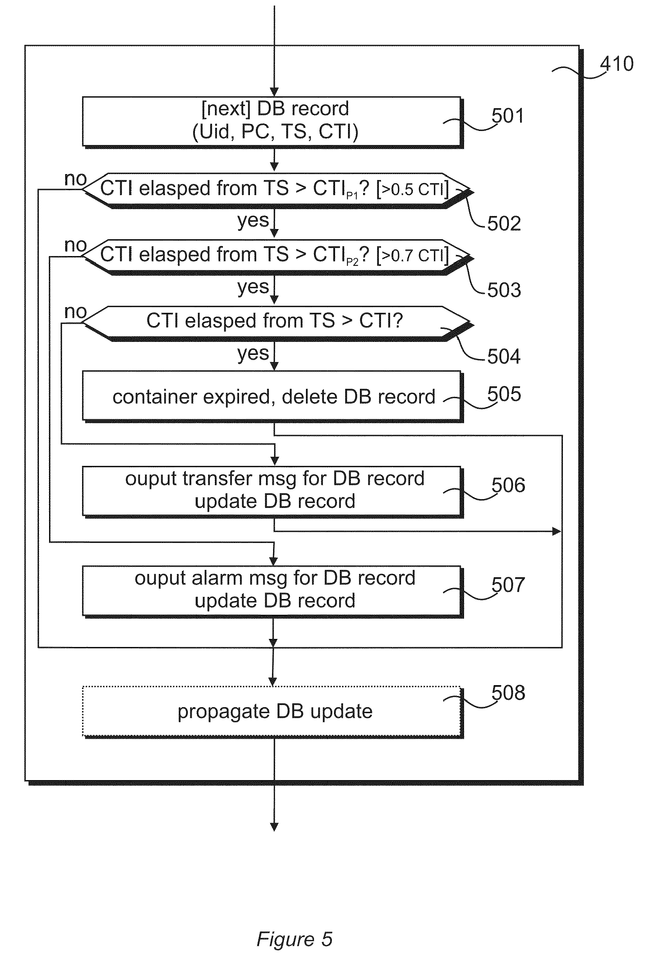

The monitoring step 410 is described hereafter in further details with reference to Figure 5 and, like the method presently described in relation to Figure 4, comprises an iterative data processing loop, which eventually arrives at a question at step 410, as to whether a next container has been presented to the RFID reader 105 interfaced with the terminal 106, for instance a further new blood container 103 for registration into the system 300. When the question of step 410 is answered positively, control returns to step 404 at which the RFID tag 104 of the further container is again read and the unique identifier thereof decoded as previously described, and so on and so forth. Alternatively, when the question of step 410 is answered negatively, control returns to the monitoring step 410.

With reference to Figure 5, a simplified representation of the data processing sub-steps performed by the system 300 for monitoring stored blood packs 103 at step 410 is now provided.

A database record as generated at step 409 is selected at step 501. A first question is asked at step 502, as to whether the period of time elapsed since the temporal reference exceeds a first sub-period of the core time interval, for instance half of the core time interval, or 18 days with reference to the example 35 days used for a blood pack 103 containing whole blood. If the question of step 502 is answered positively, a next question is asked at step 503, as to whether the period of time elapsed since the temporal reference exceeds a second, longer sub-period of the core time interval, for instance two thirds of the core time interval, or 24 days with reference to the example 35 days used for a blood pack 103 containing whole blood. If the question of step 503 is answered positively, a next question is asked at step 504, as to whether the period of time elapsed since the temporal reference exceeds the core time interval, for instance 36 days with reference to the example 35 days used for a blood pack 103 containing whole blood.

When the question of step 504 is answered positively, the blood contained in the blood pack 103 has effectively expired and many not be safely or efficiently used for a transfusion, whereby the monitoring module 302 declares the expiry and deletes the corresponding database record for avoiding its needless monitoring at step 505. Control proceeds to an optional step 508 at which the monitoring module 302 may generate a relevant container disposal message and propagate the corresponding database update to remote databases 303 stored and processed at remote terminals 106, to ensure that the expired blood pack 103 is not still registered thereat, nor mistakenly used if the expiry event occurred during its transfer.

Alternatively, when the question of step 504 is answered negatively, the blood pack 103 is still in date for use, albeit with only a limited period of useful life available , i.e. a third or less, and control proceeds to step 506, at which the monitoring module 302 generates a transfer message for transferring the container to an alternative facility 101_{N} for use, for instance the regional hospital 101₁ at which it is relatively sure to be used soonest compared to other facilities with lesser standing blood requirements. Control again proceeds to an optional step 508 at which the monitoring module 302 may propagate a corresponding database update to remote databases 303 stored and processed at remote terminals 106, informing about the forthcoming transfer of the blood pack 103.

When the question of step 503 is answered negatively, the blood pack 103 is still in date for use, however it has now reached half of its useful life and its use should be prioritised relative to stored blood packs 103 with a more recent temporal reference. Control accordingly proceeds to step 507, at which the monitoring module 302 generates an alert message for prioritising use of the container at the facility at which it is currently stored, for instance the smaller hospital 101₁ or the surgery 101₃. Failure to use the prioritised blood pack 1034 would eventually result in answering the question of step 503 positively and result in transfer of the blood pack 103 to the regional hospital 101₁ as previously described. Control again proceeds to an optional step 508 at which the monitoring module 302 may propagate a corresponding database update to remote databases 303 stored and processed at remote terminals 106, informing about the prioritised use of the blood pack 103.

When the question of step 502 is answered negatively, the blood pack 103 is still in date for use and early in its useful life, whereby its use does not require prioritising yet, nor is its transfer required. Accordingly, control proceeds to optional step 508, at which the monitoring module 302 may propagate a corresponding database update to remote databases 303 stored and processed at remote terminals 106, to inform about the status of the blood pack 103.

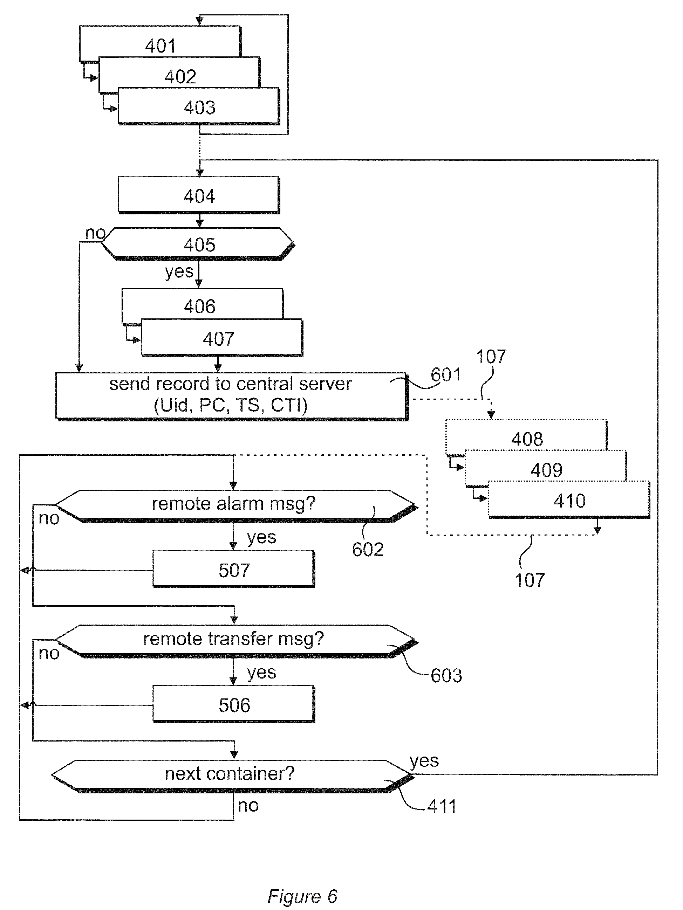

With reference now to Figure 6, a simplified representation of the data processing steps performed by the system 300 for storing, registering and monitoring blood packs 103 is provided according to a second embodiment, wherein the central server 106 determines a core predetermined time interval for each blood pack 103 stored at each remote facility 101_{N} and monitors all blood packs 103 stored thereat. In Figure 6, like data processing steps of Figures 4 and 5 are referenced by like numerals for purposes of clarity and simplicity.

In this embodiment, the monitoring function is devolved to a central server 106 remote from the healthcare facilities 101_{N}, the terminals 106 at each of which are configured with a simplified sub-system 301 consisting of at least the user interface module 307, the communication module 308 and an RFID reader Application Programmer Interface (API) interfacing the terminal 106_{N} with a local RFID reader 105, sufficient in functional terms to generate and process new blood packs 103 at steps 401, 402, 403, which are next presented for registration at steps 404, 411 and product characteristic and temporal reference for which are input at steps 406, 407, all as before.

Subsequently to inputting the temporal reference data at step 407, in this embodiment the terminal 106 communicates the unique identifier, product characteristic and temporal reference to the central server over the network 107 at step 601. The monitoring module 302 at the server invokes the rule set module 304 to look up and calculate the core time interval for the input product characteristic at step 408; at step 409 again it creates a new database record in the central database 303 for the new unique identifier, again including the unique identifier, the product characteristic, the temporal reference, the determined core time interval, and in this embodiment a storage location identifier; and control proceeds to the monitoring step 410, all as before but here performed centrally for all storage facilities 101_{N} by the central server 106.

The question of step 411 is not asked at the central server, rather a first question is asked at step 602, as to whether an alarm message has been received from the central server pursuant to steps 501 to 503 and 507 performed by the monitoring module 302 thereat, and identifying the message recipient terminal 106 of the storage facility with the additional storage location identifier. When the question of step 602 is answered positively, the local user interface module 307 outputs a corresponding alarm or prioritisation message for a blood pack 103 stored locally at step 507. Control thereafter proceeds to step 411 at the local terminal 106.

Alternatively, the question of step 602 is answered negatively and a second question is asked at step 603, as to whether a transfer instruction has been received from the central server pursuant to steps 501 to 504 and 506 performed by the monitoring module 302 thereat, and identifying the message recipient terminal 106 of the storage facility with the additional storage location identifier. When the question of step 603 is answered positively, the local user interface module 307 outputs a corresponding transfer order message for a blood pack 103 stored locally at step 506. Control thereafter proceeds to step 411 at the local terminal 106. Alternatively, the question of step 603 is answered negatively and control proceeds directly to the question of step 411 at the local terminal 106.

In this embodiment still, when the question of step 411 is answered positively, control proceed to step 404 as before, however when the question of step 411 is answered negatively, control returns to the question of step 602, such that the local terminal 106 effectively enters a network-listening mode and awaits a next network message from the remote central server.

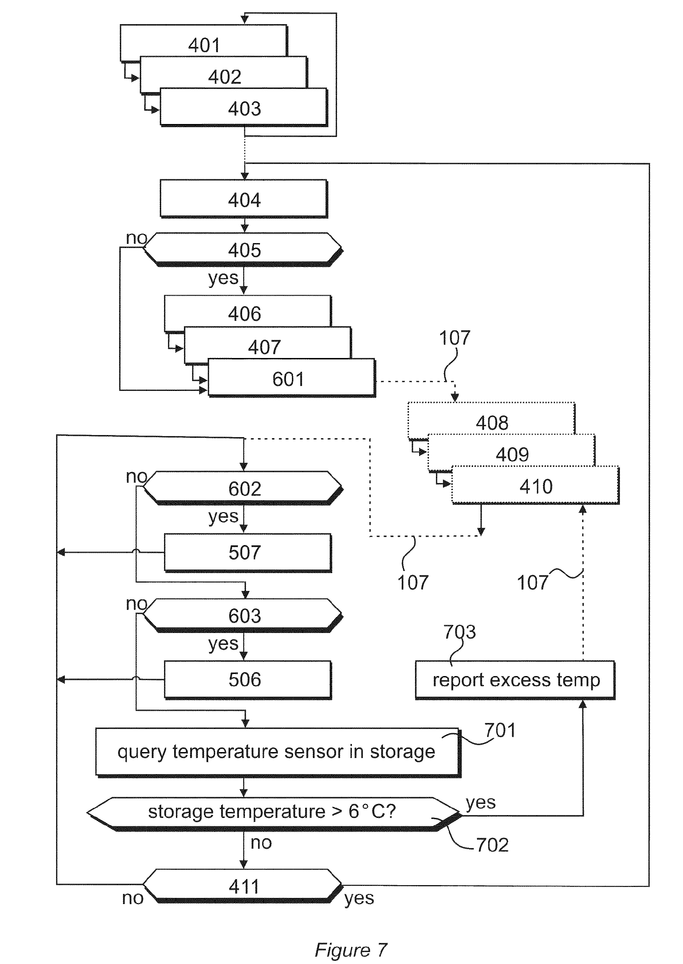

With reference now to Figure 7, a simplified representation of the data processing steps performed by the system 300 for storing, registering and monitoring blood packs 103 is provided according to a third embodiment, wherein the central server 106 again determines a core predetermined time interval for each blood pack 103 stored at each remote facility 101_{N} and monitors all blood packs 103 stored thereat, and wherein an environmental variable, in the example a storage temperature, is monitored at one or more remote facility 101_{N} and reported to the central server 106 across the network. In Figure 7, like data processing steps of Figure 6 are referenced by like numerals for purposes of clarity and simplicity.

In this embodiment, the monitoring function is again devolved to the central server 106 remote from the healthcare facilities 101_{N,} the terminals 106 at each of which are again configured with a simplified sub-system 301 as previously described, and also a temperature sensor API interfacing the terminal 106_{N} with a local temperature measuring device.

When both the questions of step 602 and step 603 are answered negatively, however before the data processing loop proceeds to the iteration query of step 411, the local temperature measuring device is queried for a temperature reading at step 701, then a question is asked at step 702, as to whether the temperature in the blood pack storage exceeds nominal parameter or range, for example the 1 to 6 °C range deemed acceptable for blood storage purposes.

When the question of step 702 is answered negatively, the temperature remains acceptable and control proceeds to the question of step 411 as described with reference to Figure 6. When the question of step 702 is answered positively, however, the temperature is excessive and likely to prejudice the quality of the stored blood packs 103. The temperature reading is thus reported to the monitoring module 302 at the central server at step 703, wherein it is processed within a suitably modified monitoring step logic, pursuant to which the respective core time interval for each stored blood pack 103 affected by the variation, jointly identified by the storage facility identifier, are decremented proportionally to the temperature increase. The local terminal 106 which has reported the temperature increase may thus eventually receive alarm messages 507 and/or transfer messages 506 sooner in respect of all its blood packs 103 stored at the time of the temperature increase, relative to the original, rule set module-provided core time interval.

The embodiments in the invention described with reference to the drawings generally comprise a computer apparatus and/or processes performed in a computer apparatus. However, the invention also extends to computer programs, particularly computer programs stored on or in a carrier adapted to bring the invention into practice. The program may be in the form of source code, object code, or a code intermediate source and object code, such as in partially compiled form or in any other form suitable for use in the implementation of the method according to the invention. The carrier may comprise a storage medium such as ROM, e.g. CD ROM, or magnetic recording medium, e.g. a floppy disk or hard disk. The carrier may be an electrical or optical signal which may be transmitted via an electrical or an optical cable or by radio or other means.

In the specification the terms "comprise, comprises, comprised and comprising" or any variation thereof and the terms include, includes, included and including" or any variation thereof are considered to be totally interchangeable and they should all be afforded the widest possible interpretation and vice versa.

The invention is not limited to the embodiments hereinbefore described but may be varied in both construction and detail.

## Claims

1. A method of monitoring a perishable product, comprising the steps of :
encoding (401) a respective unique identifier in one ore more contactless data transmitters (104);
securing (402) each encoded contactless data transmitter to a respective container (103) adapted to contain the perishable product ;
filling (403) one or more containers with the perishable product ;
storing the one or more filled container in at least one facility (101);
for each filled container, associating (409), with at least one data processing terminal (106), data defining at least one characteristic of the perishable product filling the container and at least one temporal reference for the container-filling step with the unique identifier; and
monitoring (410), at the at least one terminal, movement and/or use of each filled container over a respective predetermined time interval elapsing from the at least one temporal reference by reading the unique identifier from the contactless data transmitter.

2. The method according to claim 1, wherein the at least one terminal is a first network-connected terminal (106), and wherein the step of associating comprises the further steps of inputting the at least one characteristic of the perishable product (406) and the at least one temporal reference (407) at a second network-connected data processing terminal (106₁) remote from the first terminal, and communicating (601) the input data with the unique identifier from the second terminal to the first terminal over the network.

3. The method according to claim 1 or 2, wherein the step of monitoring comprises the further steps of periodically querying the respective contactless data transmitter of one or more containers stored in the at least one facility and updating a database at the at least one terminal.

4. The method according to any of claims 1 to 3, comprising the further step of causing the at least one terminal to automatically output (507) a message about a container stored in the at least one facility (101), when at least a first portion of the predetermined time interval associated with that container is reached.

5. The method according to claim 4, comprising the further step of causing the at least one terminal to automatically output a container transfer message (506) for transferring the container to an alternative facility (101₁) for use, when a second portion of the predetermined time interval associated with that container is reached.

6. The method according to claim 5, wherein the predetermined time interval is bounded by the temporal reference and a use-by-date, the second portion of the predetermined time interval is reached before the use-by-date, and the first portion of the predetermined time interval is reached before the second portion.

7. The method according to any of claims 1 to 6, wherein the perishable product is blood, the at least one characteristic is a blood type and the at least one temporal reference is a blood collection date.

8. A system (300) for storing a perishable product, comprising :
one or more contactless data transmitters (104), each encoded with a respective unique identifier ;
one or more containers (103), each adapted to be filled with and to contain a perishable product, and to the or each of which an encoded contactless data transmitter is respectively secured ;
storage means of at least one facility (101) for storing the or each filled container ;
means for associating (302, 303) data defining at least one characteristic of the perishable product contained in a container and at least one temporal reference for the container-filling operation with the unique identifier; and
monitoring means (302) for monitoring movement and/or use of each filled container over a respective predetermined time interval elapsing from the at least one temporal reference ; and
means for reading (105) the unique identifier from the contactless data transmitter.

9. The system according to claim 8, wherein the means for associating comprises a first networked-connected data processing terminal (106₁), the monitoring means comprises a second network-connected data processing terminal (106) remote from the first terminal, and wherein the first terminal is configured to input the at least one characteristic of the perishable product and the at least one temporal reference, and to communicate the input data with the unique identifier to the second terminal over the network.

10. The system according to claim 8 or 9, wherein the reading means (105) is further adapted to periodically query the respective contactless data transmitter of one or more containers stored in the at least one facility.

11. The system according to any of claims 1 to 3, wherein the monitoring means (302) is further configured to automatically output a respective message (507) about a container stored in the at least one facility, when at least a first portion of the predetermined time interval associated with that container is reached.

12. The system according to claim 11, wherein the monitoring means (302) is further configured to automatically output a container transfer message (506) for transferring the container to an alternative facility (101₁) for use, when a second portion of the predetermined time interval associated with that container is reached.

13. The system according to any of claims 8 to 12, wherein the predetermined time interval is bounded by the temporal reference and a use-by-date, the second portion of the predetermined time interval is reached before the use-by-date, and the first portion of the predetermined time interval is reached before the second portion.

14. The system according to any of claims 1 to 6, wherein the contactless data transmitter (104) is selected from the group comprising radiofrequency identification (RFID) passive tags and labels, battery-assisted passive RFID tags and labels, RFID active tags and labels, RuBee tags and Memory Spot tags.

15. A set of instructions recorded on a data carrying medium which, when read from the medium and processed by a data processing terminal operably connected with a network, configures the terminal to
associate data defining at least one characteristic of a perishable product filling a container stored in at least one facility and at least one temporal reference for the container-filling operation with a unique identifier encoded in a contactless data transmitter secured to the container , and
monitor movement and/or use of the container over a respective predetermined time interval elapsing from the at least one temporal reference and
read or receive the unique identifier from each contactless data transmitter.
